# EUROPEAN PATENT SPECIFICATION

(11) **EP 3 483 278 B1**
(45) Date of publication and mention of the grant of the patent: **17.06.2020**
(21) Application number: 18205242.3
(22) Date of filing: 08.11.2018
(51) Int. Cl.: C12P 5/02, C12M 1/00, C12M 1/107, B01D 53/14

(54) **METHOD FOR REUSING COMPONENTS OF DISUSED OIL FACILITIES FOR BIOGAS PRODUCTION**
VERFAHREN ZUR WIEDERVERWENDUNG VON KOMPONENTEN STILLGELEGTER ÖLANLAGEN FÜR DIE BIOGAS-PRODUKTION
PROCÉDÉ DE RÉUTILISATION DE COMPOSANTS D'INSTALLATIONS DE PÉTROLE MISES AU REBUT POUR LA PRODUCTION DE BIOGAZ

(30) Priority: 14.11.2017 IT 201700129629
(43) Date of publication of application: 15.05.2019
(73) Proprietor: Water & Soil Remediation S.r.l., 46010 Curtatone, Frazione Levata MN (IT)
(72) Inventor: PRANDI, Alberto, 46100 MANTOVA (IT)
(74) Representative: Modiano, Micaela Nadia

(56) References cited:
- WO-A1-2014/033762
- DE-B3-102005 051 952
- YANG, L. & LI, Y.: "Biogas Cleaning and Upgrading Technologies", , 26 March 2014 (2014-03-26), pages 1-6, XP002783239, Retrieved from the Internet: URL:https://ohioline.osu.edu/factsheet/AEX -653.1-14 [retrieved on 2018-07-19]

## Description

The present invention relates to a method for recovering components of disused oil facilities.

It is known that in Europe and in the rest of the world, several cases have occurred in the last decade of oil facilities, such as oil refineries and petrochemical facilities, falling into disuse.

As is known, in these facilities there are several storage tanks, both fixed-roof and floating-roof, as well as gas scrubbing plants the function of which is to remove hydrogen sulfide (H2S), also known in the jargon as sulfurated hydrogen, from the fuel gas destined for oil refinery furnaces, by contact in countercurrent with slightly alkaline aqueous solutions.

In particular, a typical gas scrubbing plant inserted in an oil refinery usually has an absorption column which is provided, at the bottom, with an inlet for the fuel gas produced by various refinery plants and constituted by a mixture of methane, ethane, propane, butane with variable concentrations of sulfurated hydrogen. The fuel gas travels with an ascending flow along the absorption column, encountering a descending flow of an alkaline solvent, and exits from said absorption column scrubbed, i.e., without the sulfurated hydrogen.

The alkaline solvent enriched with sulfurated hydrogen and in output from the absorption column is preheated in an adapted heat exchanger and sent to a regeneration column, in which it undergoes further heating, by way of circulating an adapted hot fluid in the bottom of such column, so as to cause the breakdown of the bond between the sulfurated hydrogen, which is made to exit from the top of the regeneration column, and the solvent, which, still hot, exits from the bottom of the regeneration column and is cooled in the heat exchanger in order to be reused later in the absorption column.

With respect to the typical gas scrubbing plant, described above, plant variations customized for individual production situations are adopted in industrial practice. There may be, for example, different methods of delivering heat to the bottom of the regeneration column, and additional heat exchangers and refrigeration elements, filters, coalescers and condensate separators, and so forth.

It should be noted, moreover, that oil refineries have self-production of electric power, by way of gas turbines coupled to electric generators, generally known as turbogas units, and/or steam turbines coupled to electric generators.

One greatly felt problem is to find, for various components of disused oil facilities, uses that offer an alternative to their complete demolition and their disposal.

It is also known that currently the production of biomethane is carried out in plants that are built specifically or in plants obtained by way of upgrading existing plants for the production of biogas, depending on the raw material for which they are specifically designed.

Conceptually, these plants have a fermentation part or section in which they induce, in the biomasses being processed, fermentations aimed at producing methane, with the simultaneous forming of carbon dioxide.

These fermentations, known in the jargon as anaerobic digestions, occur in tanks, known as fermenters, which are specifically built and inside which a gaseous mixture is generated which is composed mainly of methane and carbon dioxide at approximately equal concentrations and with the presence of other gases at lower concentrations, their generation depending on the type and composition of the raw material.

In its general outline, the fermentation part of a biomethane production plant is identical to the one that is present in a biogas production plant, in which, in particular, the partial transformation occurs of the biomass into a mixture of methane and carbon dioxide, known in fact as biogas, again inside tanks, also known as fermenters, which are built specifically to contain both a liquid phase, together with the fermenting slurry, and a gaseous phase which develops progressively.

Biogas is normally used to supply spark-ignition internal combustion engines coupled to a corresponding number of electrical generators, in which the carbon dioxide passes through the combustion chambers without undergoing transformation, while the methane burns, and is also converted to carbon dioxide.

Biomethane production plants represent an evolution of biogas plants, since the carbon dioxide generated in the fermenter is separated from the methane, which can thus be used pure in various applications, for example compressed in cylinders (CNG) for vehicle propulsion, liquefied (LNG) for heavy transport, and introduced into the methane network for domestic use, industrial use and various other uses.

The operation of separating the carbon dioxide from the methane is known in the jargon as "upgrading" the biogas to produce biomethane.

In the most widespread applications, the separation of the carbon dioxide from the methane is carried out by way of processes with semipermeable membranes or by way of processes with molecular sieves or also by way of processes with pressurized water.

The first of these processes is based on the principle of selective permeation of one gas with respect to the other through a membrane, the second is based on the principle of differentiated adsorption of one gas with respect to the other on a zeolite substrate, for which there is also the variation under vacuum conditions, known as VPSA (vacuum pressure swing adsorption), while the third utilizes the higher solubility in water of pressurized carbon dioxide with respect to methane.

A fourth process for separating carbon dioxide from methane uses a chemical reaction between the carbon dioxide, which is a slightly acid compound, and a compound with a slightly alkaline reaction, which is typically constituted by an organic amine and sequesters the carbon dioxide from the biogas, allowing the current of purified methane to flow, in order to then undergo regeneration by heating, with release of the previously acquired dioxide.

Although it is known, this fourth process has not yet found widespread application, since it is challenging in terms of investment cost with respect to the modest size of most biogas/biomethane production plants.

The process most widely used currently in Italy is the one with semipermeable membranes. However, this process, like the one with molecular sieves, has severe limitations both owing to the high electrical consumption, requiring the compression of the entire biogas mixture and consequently also of the carbon dioxide, and owing to the significant losses of methane, which is left in the current of carbon dioxide.

Moreover, the methane fraction contained in the carbon dioxide is no longer recoverable and, when released into the atmosphere, produces a greenhouse effect with a considerably greater intensity than carbon dioxide, in addition to being an overall loss of precious energy.

The patent document WO 2014/033762 A1 (ENI S.P.A., 6 March 2014) discloses a method for recovering components of disused oil facilities, said facilities comprising:
two hydrodesulfurization units,
characterized in that the method provides for
the reuse of said two hydrodesulfurization units to provide a liquid fuel production plant using triglycerides.

The aim of the present invention is to provide a method for recovering components of disused oil facilities that is capable of improving the background art in one or more of the aspects indicated above.

Within this aim, an object of the invention is to provide a method for recovering components of disused oil facilities that makes it possible to overcome at least partly the drawbacks linked to the high investment costs of a biomethane production plant.

Another object of the invention is to offer a solution also to the drawbacks linked to the significant losses of biomethane in the biogas purification process.

Another object of the invention is to provide a method for recovering components of disused oil facilities that is relatively easy to provide and can be carried out at low cost.

This aim, as well as these and other objects which will become better apparent hereinafter are achieved by a method for recovering components of disused oil facilities according to claim 1, optionally provided with one or more of the characteristics of the dependent claims.

The invention relates to a method for recovering components of disused oil facilities, said facilities comprising:
at least one oil product storage tank and
at least one gas scrubbing plant, provided with at least one absorption column (2), provided with a fuel gas intake duct (1), with a duct (4) for supplying a solvent, with a discharge duct (3) for the scrubbed gas and with an outflow duct (5) for the enriched solvent, and at least one regeneration column (7), which is adapted to receive in input the enriched solvent that arrives from said outflow duct (5) and is provided with heating means, a duct (11) for the extraction of the regenerated solvent, connected to said supply duct (4), and a duct (9) for the evacuation of the substances separated from the solvent,
characterized in that the method provides for
the reuse of at least part of said at least one tank to provide at least one biomass fermenter in order to generate biogas and
the reuse of at least part of said gas scrubbing plant to provide a biogas purification plant in order to obtain biomethane.

Further characteristics and advantages of the invention will become better apparent from the description of a preferred but not exclusive embodiment of the method according to the invention, which is illustrated by way of example in the accompanying drawings, wherein:
Figure 1 is a schematic view of a gas scrubbing plant typically used in oil refineries;
Figure 2 is a diagram of a plant that can be obtained by way of the method according to the invention;
Figure 3 is a schematic view of an alternative example of a plant that can be obtained by way of the method according to the invention.

With reference to the figures, the method for recovering components of disused oil facilities, according to the invention, is applied in particular to disused oil facilities, such as oil refineries or petrochemical facilities, which comprise one or more storage tanks and one or more gas scrubbing plants.

In particular, according to the method of the present invention, at least a part of the storage tanks, which were previously used to store the oil products, is reused as fermenters to produce, from the fermentation of biomasses introduced as raw material, a biogas mainly constituted by biomethane and carbon dioxide, while at least a part of the gas scrubbing plants, the function of which in oil facilities was to remove the hydrogen sulfide or sulfurated hydrogen contained in the fuel gas destined for the oil refinery furnaces, is recovered in order to treat the biogas produced in the fermenters, removing the carbon dioxide from the biomethane.

Conveniently, the fermentable raw material that is loaded into the fermenter obtained from the reuse of preexisting storage tanks can be constituted by second- and/or third-generation biomasses, substances derived from the pretreatment of second- and/or third-generation biomasses, substances derived from the saccharification of second- and/or third-generation biomasses, and the like.

The storage tanks that can be recovered by means of the proposed method can be of the fixed-roof or floating-roof type.

In practice, the containment structure, i.e., the side wall and the bottom, of the storage tanks that already exist in the disused oil facility is maintained and reused in order to provide the chamber for the fermentation of the biomass in input, in which microorganisms are inoculated which carry out the fermentation process.

It should be noted that for fixed-roof tanks it is possible to provide simple adaptations to the new function as fermenters, such as for example the application of one or more internal agitators and the installation at the roof, or, in any case, at the top of the tank of a suitable extraction pipe, for the collection and exit of the gaseous phase constituted by the biogas that is generated during the fermentation.

For floating-roof tanks, for their adaptation to the function as fermenters, the corresponding floating roofs are instead removed and replaced with fixed roofs; as an alternative, after the removal of the floating roof, it is possible to provide advantageously for the application of means adapted to contain the gaseous phase constituted by the biogas being formed, which can be constituted conveniently by a flexible internal membrane, the function of which is to contain both the liquid phase and the gaseous phase within the resulting fermenter and which is provided with a manifold or with a pipe for extracting the biogas at its top.

It is also possible to provide for the application of an elastic membrane provided with an extraction pipe which is installed only at the top of the existing storage tank, so as to obtain a fermenter constituted by the side wall and by the bottom of the preexisting tank and by a new ceiling provided by the installed elastic membrane.

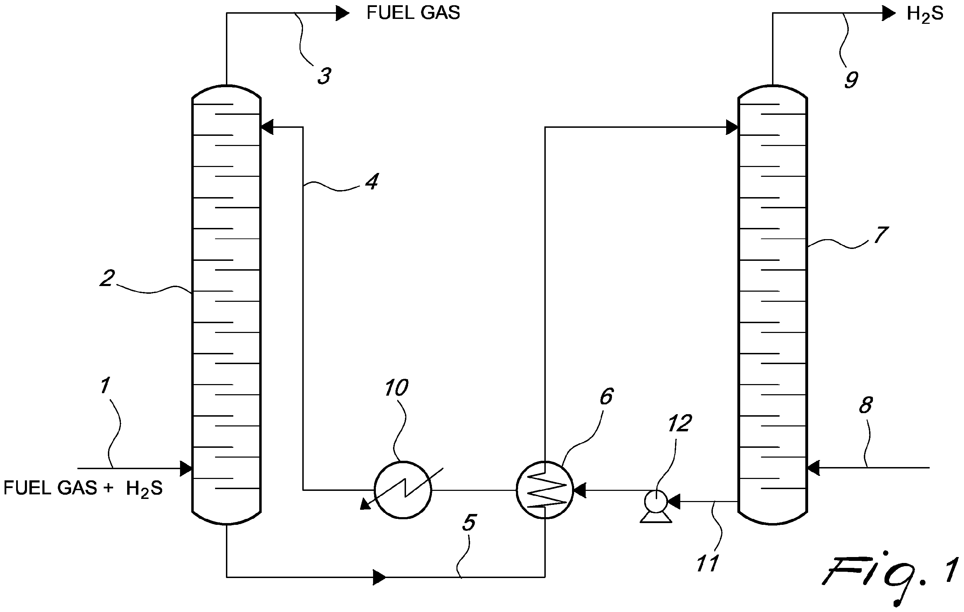

Figure 1 shows the typical diagram of a gas scrubbing plant of a common oil refinery, which according to the proposed method can be reused as a methane purification plant or as a plant for removing carbon dioxide from biogas.

In particular, in the typical gas scrubbing plant, the fuel gas produced by various refinery plants and constituted by a mixture of methane, ethane, propane, butane and containing variable concentrations of sulfurated hydrogen, enters, through an intake duct 1, the lower part of an absorption column 2, travels with an ascending flow along the absorption column 2, encountering a descending flow of alkaline solvent introduced into the absorption column 2, through a supply duct 4, and exits, through a discharge duct 3, from this absorption column 2 scrubbed, i.e., without the sulfurated hydrogen. The solvent enriched with sulfurated hydrogen exits from the absorption column 2 through an outflow duct 5, is conveniently heated in a preheating heat exchanger 6 and enters a regeneration column 7, where an additional application of heat provided by heating means, for example by way of circulation in the bottom of the regeneration column 7 of a hot fluid that arrives from a feed duct 8, causes the breakdown of the solvent-sulfurated hydrogen bond. The sulfurated hydrogen, thus separated from the solvent, exits from the regeneration column head 7 by way of an evacuation duct 9. The regenerated solvent exits hot from the bottom of the regeneration column 7 by way of an extraction duct 11 and is sent, by way of a recirculation pump 12, into the preheating exchanger 6, where it transfers heat to the solvent enriched with sulfurated hydrogen entering the regeneration column 7, before it is further cooled in a heat exchanger 10 and returns, through the supply duct 4, into the absorption column 2.

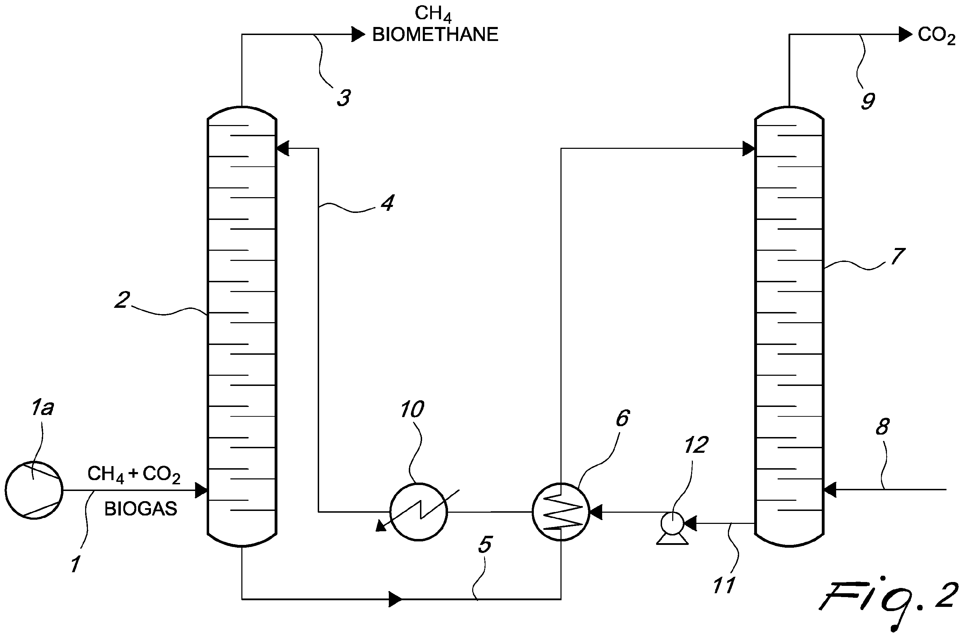

Figure 2 shows that the same apparatuses of a preexisting gas scrubbing plant can be reused to obtain a methane purification plant that allows the removal of carbon dioxide from the biogas produced by a fermenter, obtained by reusing a preexisting storage tank, or more generally by a biogas production plant.

In particular, the biogas produced by fermentation and constituted by a mixture of methane and carbon dioxide and optionally containing slight concentrations of sulfurated hydrogen is made to enter, through the intake duct 1 which is conveniently connected to the extraction pipe of the fermenter, at the bottom of the absorption column 2.

It should be noted that conveniently the biogas is introduced into the intake duct 1 after being compressed. The fermenter in fact produces biogas at atmospheric pressure, while the scrubbing column 2 operates validly at a pressure higher than atmospheric pressure, for example between a minimum of 1.1 absolute bar and a maximum of 5 absolute bar. Therefore, advantageously a compressor 1a is installed along the intake duct 1 and makes it possible to introduce the biogas into the intake duct 1 at an adequate pressure level.

The biogas thus supplied flows through the absorption column 2 in an ascending flow, encountering a descending flow of alkaline solvent, which is introduced through the supply duct 4, and then exits from the top of the absorption column 2, through the discharge duct 3, scrubbed, i.e., deprived of the carbon dioxide and of any sulfurated hydrogen, so as to be constituted substantially by practically pure methane or biomethane.

The solvent enriched with carbon dioxide and possibly with sulfurated hydrogen instead exits from the outflow duct 5 of the absorption column 2 and is heated in the preheating heat exchanger 6 before entering the regeneration column 7, in which, by way of the input of heat provided by the hot fluid that enters at the bottom of the regeneration column 7, through the feed duct 8, the breakdown of the solvent-carbon dioxide bond and optionally of the solvent-sulfurated hydrogen bond is caused.

The carbon dioxide, together with the sulfurated hydrogen, if present in the initial biogas, then exits from the head of the regeneration column by way of the evacuation duct 9, while the regenerated solvent exits hot from the bottom of the regeneration column through the extraction duct 11 and is sent by the recirculation pump 12 into the preheating exchanger 6, where it transfers heat to the solvent enriched with carbon dioxide entering the regeneration column 7, before undergoing a further cooling in the heat exchanger 10 and reaching the absorption column 2 by way of the supply duct 4 for a subsequent absorption cycle.

It should be noted that the alkaline solvent that can be used to remove the carbon dioxide from the biogas can be the same solvent used previously in the gas scrubbing plant to remove sulfurated hydrogen from fuel gas, although it is preferable to use a more bland alkaline substance in order to save energy in the regeneration column 7.

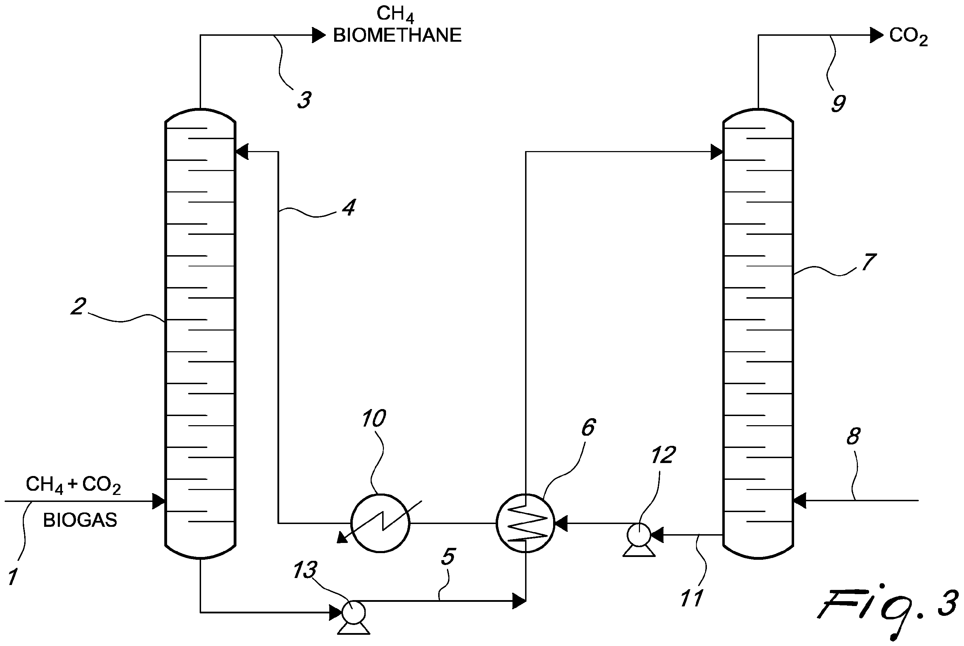

It should be noted that it is also possible to provide for the absorption column 2 to be able to work at atmospheric pressure. In this case, the absorption column 2 can be connected directly downstream of the fermenter that produces biogas at atmospheric pressure, therefore without the need to install the compressor 1a in input to the absorption column 2. Also in this case, as shown in Figure 3, a transfer pump 13 is advantageously installed along the outflow duct 5 and makes it possible to send the alkaline solution rich in carbon dioxide into the regeneration column 7.

Optionally, as mentioned above, with respect to the typical gas scrubbing plant described above, the gas scrubbing plants that are present in individual disused oil facilities can have different plant variations depending on the requirements. There can be, for example, different methods of supplying heat at the bottom of the regeneration column 7, in addition to further heat exchangers and coolers, filters, coalescers and condensate separators and the like.

In any case, apart from the above mentioned variations, the gas scrubbing plants derived from disused oil facilities which, according to the method of the present invention, are reused in order to obtain a plant for purifying the methane produced by biogas plants with minimal investment cost, have the basic layout shown in Figure 1.

It should be noted that the gas scrubbing plant, in its new function as methane purification plant, will operate at significantly lower pressures than the pressures to which it was previously subjected in the oil facility, with a consequent major energy saving linked to the lack of a need to compress the biogas to be treated.

It should be noted, furthermore, that the method according to the invention provides conveniently for the reuse of preexisting turbogas units and of generators that were previously driven by steam, which can instead be coupled to gas turbines.

It should be noted furthermore that as regards the corrosiveness of the processes, the new conditions envisaged for the plants obtained with the method according to the present invention are generally less corrosive than typical oil plant conditions, and are such as to maintain the validity of the existing metallurgies.

In practice it has been found that the invention makes it possible to achieve the intended aim and objects, since it offers the possibilityto recover components of disused oil facilities, which otherwise would remain abandoned or would be dismantled in order to be disposed of or recycled as waste, thus providing, with extremely modest costs, plants for producing biogas and/or biomethane.

It is estimated, in particular, that the method according to the invention makes it possible to save an investment portion of the order of 70% with respect to the investment cost required for the construction of new fermenters and new biomethane purification plants.

Another advantage of the invention is constituted by the fact that the summation of the consumptions and losses in the methane purification plant obtained according to the method of the present invention are much lower than those of membrane processes or molecular sieve processes.

In practice, the materials used, so long as they are compatible with the specific use, as well as the contingent shapes and dimensions, may be any according to the requirements and the state of the art.

Where technical features mentioned in any claim are followed by reference signs, those reference signs have been included for the sole purpose of increasing the intelligibility of the claims and accordingly such reference signs do not have any limiting effect on the interpretation of each element identified by way of example by such reference signs.

## Claims

1. A method for recovering components of disused oil facilities, said facilities comprising at least one oil product storage tank and at least one gas scrubbing plant, provided with at least one absorption column (2), provided with a fuel gas intake duct (1), with a duct (4) for supplying a solvent, with a discharge duct (3) for the scrubbed gas and with an outflow duct (5) for the enriched solvent, and at least one regeneration column (7), which is adapted to receive in input the enriched solvent that arrives from said outflow duct (5) and is provided with heating means, a duct (11) for the extraction of the regenerated solvent, connected to said supply duct (4), and a duct (9) for the evacuation of the substances separated from the solvent, **characterized in that** the method provides for the reuse of at least part of said at least one tank to provide at least one biomass fermenter in order to generate biogas and the reuse of at least part of said gas scrubbing plant to provide a biogas purification plant in order to obtain biomethane.

2. The method according to claim 1, **characterized in that** the reuse of at least part of said at least one tank provides for keeping the containment structure of said at least one tank in order to provide a fermentation chamber, applying an extraction pipe to the top of said tank, and loading a fermentable biomass into said at least one tank in order to generate, by way of fermentation of said biomass, biogas which exits through said extraction duct.

3. The method according to one or more of the preceding claims, **characterized in that** said at least one tank comprises a fixed-roof tank.

4. The method according to one or more of the preceding claims, **characterized in that** it provides for the application of at least one internal agitator in said at least one tank.

5. The method according to claim 2, **characterized in that** said at least one tank comprises at least one floating-roof tank, said method providing for the removal of said floating roof and the application to said at least one tank of means adapted to contain the biogas.

6. The method according to claim 5, **characterized in that** said means adapted to contain biogas comprise a flexible internal membrane, the function of which is to contain both the liquid phase and the gaseous phase, said membrane being provided at the top with said biogas extraction pipe.

7. The method according to claim 5, **characterized in that** said means adapted to contain biogas comprise a flexible membrane provided with said extraction pipe and installed only at the top of said tank.

8. The method according to one or more of the preceding claims, **characterized in that** the reuse of said gas scrubbing plant provides for supplying said intake duct (1) with a biogas obtained by fermentation of fermentable raw material, instead of said fuel gas, and introducing into said absorption column (2) through said supply duct (4) an alkaline solvent adapted to remove mainly carbon dioxide, instead of an alkaline solvent adapted to remove mainly sulfurated hydrogen.

9. The method according to claim 2, **characterized in that** it provides for connecting said extraction pipe to said intake duct (1).

10. The method according to one or more of the preceding claims, **characterized in that** it provides for the installation of a compressor (la) along said intake duct (1) in order to introduce said biogas into said absorption column (2) at a pressure that is higher than atmospheric pressure.

11. The method according to claim 2, **characterized in that** it provides for directly connecting said extraction pipe to said intake duct (1) in order to introduce said biogas into said absorption column (2) at atmospheric pressure and for installing a transfer pump (13) along said outflow duct (5).

## Patentansprüche

1. Ein Verfahren zur Wiederverwendung von Komponenten stillgelegter Ölanlagen, wobei die Anlagen mindestens einen Ölprodukt-Speichertank und mindestens eine Gaswaschanlage umfassen, ausgestattet mit mindestens einer Absorptionskolonne (2), versehen mit einer Brenngas-Einlassleitung (1), mit einer Leitung (4) zum Zuführen eines Lösungsmittels, mit einer Ablassleitung (3) für das gewaschene Gas und mit einer Ablaufleitung (5) für das angereicherte Lösungsmittel; und mindestens einer Regenerierkolonne (7), die ausgebildet ist, um das angereicherte Lösungsmittel, das von der Ablaufleitung (5) kommt, aufzunehmen, und mit Heizmitteln, einer Leitung (11) für die Extraktion des regenerierten Lösungsmittels, verbunden mit der Zuführleitung (4), und einer Leitung (9) für die Evakuierung der vom Lösungsmittel abgetrennten Substanzen ausgestattet ist; **dadurch gekennzeichnet, dass** das Verfahren die Wiederverwendung mindestens eines Teils des mindestens einen Tanks beinhaltet, um mindestens einen Biomasse-Fermenter bereitzustellen, um Biogas zu erzeugen; und die Wiederverwendung mindestens eines Teils der Gaswaschanlage, um eine Biogasreinigungsanlage zur Gewinnung von Biomethan bereitzustellen.

2. Das Verfahren gemäß Anspruch 1, **dadurch gekennzeichnet, dass** die Wiederverwendung mindestens eines Teils des mindestens einen Tanks Folgendes beinhaltet: das Aufbewahren der Behälterstruktur des mindestens einen Tanks, um eine Gärkammer bereitzustellen, das Anbringen eines Extraktionsrohrs an der Oberseite des Tanks und das Beladen des mindestens einen Tanks mit einer fermentierbaren Biomasse, um durch Fermentation der Biomasse Biogas zu erzeugen, das durch die Extraktionsleitung austritt.

3. Das Verfahren gemäß einem oder mehreren der obigen Ansprüche, **dadurch gekennzeichnet, dass** der mindestens eine Tank einen Festdachtank umfasst.

4. Das Verfahren gemäß einem oder mehreren der obigen Ansprüche, **dadurch gekennzeichnet, dass** es die Anwendung mindestens eines internen Rührwerks in dem mindestens einen Tank vorsieht.

5. Das Verfahren gemäß Anspruch 2, **dadurch gekennzeichnet, dass** der mindestens eine Tank mindestens einen Schwimmdachtank umfasst, wobei das Verfahren das Entfernen des Schwimmdachs und das Anbringen von Mitteln, die ausgebildet sind, um das Biogas aufzunehmen, an dem mindestens einen Tank vorsieht.

6. Das Verfahren gemäß Anspruch 5, **dadurch gekennzeichnet, dass** die Mittel, die ausgebildet sind, um Biogas aufzunehmen, eine flexible innere Membran umfassen, die die Funktion hat, sowohl die Flüssigphase als auch die Gasphase aufzunehmen, wobei die Membran an der Oberseite mit dem Biogas-Extraktionsrohr angebracht ist.

7. Das Verfahren gemäß Anspruch 5, **dadurch gekennzeichnet, dass** die Mittel, die ausgebildet sind, um Biogas aufzunehmen, eine flexible Membran umfassen, die mit dem Extraktionsrohr bereitgestellt und nur an der Oberseite des Tanks installiert ist.

8. Das Verfahren gemäß einem oder mehreren der obigen Ansprüche, **dadurch gekennzeichnet, dass** die Wiederverwendung der Gaswaschanlage Folgendes vorsieht: das Zuführen eines Biogases in die Einlassleitung (1), das gewonnen wird durch die Fermentation fermentierbaren Rohmaterials anstelle von Brenngas und das Einführen eines alkalischen Lösungsmittels, das geeignet ist, hauptsächlich Kohlendioxid zu entfernen, durch die Zuführleitung (4), anstelle eines alkalischen Lösungsmittels, das geeignet ist, hauptsächlich geschwefelten Wasserstoff zu entfernen.

9. Das Verfahren gemäß Anspruch 2, **dadurch gekennzeichnet, dass** es die Verbindung des Extraktionsrohrs mit der Einlassleitung (1) vorsieht.

10. Das Verfahren gemäß einem oder mehreren der obigen Ansprüche, **dadurch gekennzeichnet, dass** es die Installation eines Kompressors (1a) entlang der Einlassleitung (1) vorsieht, um das Biogas bei einem Druck, der höher ist als atmosphärischer Druck, in die Absorptionskolonne (2) einzulassen.

11. Das Verfahren gemäß Anspruch 2, **dadurch gekennzeichnet, dass** es die direkte Verbindung des Extraktionsrohrs mit der Einlassleitung (1), um das Biogas bei atmosphärischem Druck in die Absorptionskolonne (2) einzulassen, und das Installieren einer Umschlagpumpe (13) entlang der Ablaufleitung (5) vorsieht.

## Revendications

1. Procédé pour récupérer des composants d'installations pétrolières désaffectées, lesdites installations comprenant au moins un réservoir de stockage de produit pétrolier et au moins une unité d'épuration de gaz, dotée d'au moins une colonne d'absorption (2), dotée d'un conduit d'arrivée (1) de gaz combustible, avec un conduit (4) pour l'alimentation en un solvant, avec un conduit de décharge (3) pour le gaz épuré et avec un conduit d'évacuation (5) pour le solvant enrichi, et au moins une colonne de régénération (7), qui est adaptée pour recevoir en entrée le solvant enrichi qui arrive par ledit conduit d'évacuation (5) et est dotée d'un moyen de chauffage, un conduit (11) pour l'extraction du solvant régénéré, relié audit conduit d'alimentation (4), et un conduit (9) pour l'évacuation des substances séparées du solvant, **caractérisé en ce que** le procédé pourvoit à la réutilisation d'au moins une partie dudit au moins un réservoir pour fournir au moins un fermenteur de biomasse afin de produire du biogaz et à la réutilisation d'au moins une partie de ladite unité d'épuration de gaz pour fournir une unité de purification de biogaz afin d'obtenir du biométhane.

2. Procédé selon la revendication 1, **caractérisé en ce que** la réutilisation d'au moins une partie dudit au moins un réservoir pourvoit à maintenir la structure de retenue dudit au moins un réservoir afin de fournir une chambre de fermentation, appliquer un conduit d'extraction au sommet dudit réservoir, et introduire une biomasse fermentable dans ledit au moins un réservoir afin de produire, par fermentation de ladite biomasse, du biogaz qui est évacué par ledit conduit d'extraction.

3. Procédé selon une ou plusieurs des revendications précédentes, **caractérisé en ce que** ledit au moins un réservoir comprend un réservoir à toit fixe.

4. Procédé selon une ou plusieurs des revendications précédentes, **caractérisé en ce qu'**il pourvoit à l'application d'au moins un agitateur interne dans ledit au moins un réservoir.

5. Procédé selon la revendication 2, **caractérisé en ce que** ledit au moins un réservoir comprend au moins un réservoir à toit flottant, ledit procédé pourvoyant à l'enlèvement dudit toit flottant et à l'application sur ledit au moins un réservoir d'un moyen adapté pour contenir le biogaz.

6. Procédé selon la revendication 5, **caractérisé en ce que** ledit moyen adapté pour contenir le biogaz comprend une membrane interne flexible, dont la fonction est de contenir à la fois la phase liquide et la phase gazeuse, ladite membrane étant munie au sommet dudit conduit d'extraction de biogaz.

7. Procédé selon la revendication 5, **caractérisé en ce que** ledit moyen adapté pour contenir le biogaz comprend une membrane flexible munie dudit conduit d'extraction et installée seulement au sommet dudit réservoir.

8. Procédé selon une ou plusieurs des revendications précédentes, **caractérisé en ce que** la réutilisation de ladite unité d'épuration de gaz pourvoit à alimenter ledit conduit d'entrée (1) avec un biogaz obtenu par fermentation de matière première fermentable, au lieu dudit gaz combustible, et introduire dans ladite colonne d'absorption (2) par ledit conduit d'alimentation (4) un solvant alcalin adapté pour extraire principalement du dioxyde de carbone, au lieu d'un solvant alcalin adapté pour extraire principalement de l'hydrogène sulfuré.

9. Procédé selon la revendication 2, **caractérisé en ce qu'**il pourvoit à relier ledit conduit d'extraction audit conduit d'arrivée (1).

10. Procédé selon une ou plusieurs des revendications précédentes, **caractérisé en ce qu'**il pourvoit à l'installation d'in compresseur (la) le long dudit conduit d'arrivée (1) afin d'introduire ledit biogaz dans ladite colonne d'absorption (2) sous une pression qui est plus élevée que la pression atmosphérique.

11. Procédé selon la revendication 2, **caractérisé en ce qu'**il pourvoit à relier directement ledit conduit d'extraction audit conduit d'arrivée (1) afin d'introduire ledit biogaz dans ladite colonne d'absorption (2) sous la pression atmosphérique et à installer une pompe de transfert (13) le long dudit conduit d'évacuation (5).
